# EUROPÄISCHE PATENTSCHRIFT

(11) **EP 2 136 660 B1**
(45) Veröffentlichungstag und Bekanntmachung des Hinweises auf die Patenterteilung: **10.10.2012**
(21) Anmeldenummer: 07857498.5
(22) Anmeldetag: 12.12.2007
(51) Int. Cl.: A24F 47/00

(54) **RAUCHFREIES ZIGARETTENERSATZPRODUKT**
SMOKE-FREE SUBSTITUTE CIGARETTE PRODUCT
PRODUIT DE REMPLACEMENT D'UNE CIGARETTE EXEMPTE DE FUMÉE

(30) Priorität: 20.03.2007 EP 07104524
(43) Veröffentlichungstag der Anmeldung: 30.12.2009
(73) Patentinhaber: Wedegree GmbH, 20357 Hamburg (DE)
(72) Erfinder: RINKER, Arno, 20251 Hamburg (DE)
(74) Vertreter: Office Freylinger
(86) Internationale Anmeldenummer: PCT/EP2007/063840
(87) Internationale Veröffentlichungsnummer: WO 2008/113420

(56) Entgegenhaltungen:
- EP-A- 0 358 114
- EP-A- 0 858 744
- WO-A-02/47499
- WO-A-2006/082571
- WO-A-2007/008825
- DE-A1- 19 854 008
- US-A- 5 535 735
- US-A1- 2004 031 495

## Beschreibung

### Technisches Gebiet

Die vorliegende Erfindung betrifft allgemein ein Verfahren und eine Vorrichtung zur rauchfreien Inhalation von Nikotin und Zusatzstoffen.

### Stand der Technik

Beim Rauchen einer herkömmlichen Zigarette wird Tabak verbrannt und der bei dieser Verbrennung entstehende Rauch wird inhaliert (Hauptstromrauch) oder an die Umgebung abgegeben (Nebenstromrauch).

Der Hauptstromrauch ist maßgeblich für die Gesundheitsschädigung des Konsumenten verantwortlich, liefert ihm aber den -gewünschten Genuss. Der Nebenstromrauch ist maßgeblich für die Gesundheitsschädigung der Passivraucher verantwortlich und ist weder von den Passivrauchern noch vom Konsumenten gewünscht.

Im Rauch einer Zigarette wurden über 4800 verschiedene Substanzen nachgewiesen von denen ungefähr 70 als nachweislich krebserregend gelten.

Bis heute existiert jedoch keine ernstzunehmende Konkurrenz zur Zigarette. Die meisten bekannten Produkte sind eigentlich Nikotin-Substitute und nicht als Genussprodukte, sondern als Entwöhnungsprodukte gedacht.

Dagegen sind bekannte Produkte, die gezielt auf eine Alternative zur Zigarette ohne deren negative Aspekte hingerichtet sind, oft durch Nachteile behaftet, die deren Akzeptanz beim Raucher stark beschränken.

Gemein ist diesen Produkten, dass sie nicht die Verbrennungswärme des Tabaks, sondern andere Energiequellen zur Freisetzung eines (Rauch-)Aerosols benutzen, wodurch die Hauptquelle des Nebenstromrauchs weitgehend entfällt.

Vorrichtungen deren Substrate mittels elektrischer Energie, sei es in Form eines Heizwiderstandes, einer Induktionsheizung oder eines Ultraschallzerstäubers, verflüchtigt werden, sind bekannt, haben aber als Nachteil, dass sie wegen ihrer Größe oder ihres Gewichts in der Hand gehalten werden müssen, dass sie sehr teuer in der Herstellung sind und/oder dass sie durch ihre (Schwermetall-)Bestandteile nur schwer umweltgerecht zu entsorgen sind.

Auch wurden Produkte vorgestellt deren Energie aus brennbaren kohlenstoffhaltigen Feststoffen, wie Celluloseschäumen, Holzkohle, usw. gewonnen wird. Der Nachteil ist hierbei generell, dass ein Nebenstromrauch entsteht, wenn auch in geringeren Mengen und im Prinzip weniger mit Schadstoffen belastet, als bei herkömmlichen Zigaretten bzw. (kleinen) Zigarren.
Aus der WO 2006/082571 ist ein derartiges gasbeheiztes Produkt bekannt.

### Aufgabe der Erfindung

Eine Aufgabe der vorliegenden Erfindung ist es für den Benutzer den Rauchakt einer normalen Zigarette so vollständig wie möglich zu imitieren. Hierzu sind ein Verfahren und eine auf diesem Verfahren basierende Vorrichtung zu entwickeln. Die haptische Wahrnehmung der Vorrichtung sollte in der Hand wie am Mund durch Größe und Gewicht einer Zigarette oder (kleinen) Zigarre sehr ähnlich sein. Handhabung und Kosten der Vorrichtung dürfen keinen großen Unterschied zum Konsum herkömmlicher Zigaretten bzw. (kleiner) Zigarren aufweisen.

### Allgemeine Beschreibung der Erfindung

Diese Aufgabe wird erfindungsgemäß gelöst durch die Bereitstellung eines Verfahrens zur Verflüchtigung von Wirk- und/oder Aromastoffen zwecks Abgabe eines inhalierbaren Aerosols, wobei Verbrennungsgase eines brennbaren Gases, welches vorzugsweise im Wesentlichen mit Luftüberschuss verbrannt wird, teilweise oder vollständig, gegebenenfalls vermischt mit Umgebungsluft, durch einen Wirk- und/oder Aromastoff-Depot geführt werden und wobei eine gewünschte Temperatur durch den Anteil an Verbrennungsgasen und ggf. durch das Mischungsverhältnis dieser Verbrennungsgase mit Umgebungsluft wählbar ist.

Ein solches Verfahren, vorzugsweise zum Inhalieren einer Nikotin-Aroma-Mixtur, wird ermöglicht durch die saubere Verbrennung eines Luft-Gasgemisches, d.h. die Abgase bestehen aus Stickstoff, gesundheitlich unbedenklichem Kohlenstoffdioxid, Wasser und ggf. Restsauerstoff und können so gefahrlos inhaliert werden. Dabei ist der Vorteil eines solchen Verfahrens im Gegensatz zu Verfahren worin die Wärme ausschließlich über Wärmetauscher an die Luft übertragen wird, dass prinzipbedingt eine Vorrichtung zur Durchführung dieses Verfahrens mit sehr viel kleineren Abmessungen bei höherem Wirkungsgrad und niedrigeren Herstellungskosten zu bewerkstelligen ist.

In einer weiteren Ausgestaltung erfolgt die Einstellung des Anteils an Verbrennungsgasen, bzw. des Mischungsverhältnisses dieser Verbrennungsgase mit Umgebungsluft durch Reglung des Mengenflusses an brennbarem Gas, wobei die Reglung des Mengenflusses an brennbarem Gas vorzugsweise mittels eines durch einen Sog erzeugten Unterdrucks und/oder Luftstroms erfolgt.

Das beschriebene Verfahren wird ausgeführt mit einer erfindungsgemäßen Vorrichtung zur möglichst vollständigen Imitation des Rauchaktes einer Zigarette, umfassend
➢ ein einen Wirk- und/oder Aromastoff-Depot (32) enthaltendes Mundstück (3), vorzugsweise in Form und Abmessungen dem Filter inklusive Banderole einer Zigarette oder kleinen Zigarre ähnlich,
➢ einen Heizstab (2) zum Bereitstellen von heißem Abgas, vorzugsweise in Form und Abmessungen dem Tabakstrang einer Zigarette bzw. einer (kleinen) Zigarre ähnlich, umfassend
   eine Gehäusehülse (20) mit einem oder mehreren Lufteingängen und einem oder mehreren mundstückseitigen Warmluftausgängen,
   ein Füllventil (21) zum Befüllen
   eines Minigastanks (22) mit einem brennbaren Gas, vorzugsweise Propan- oder Butangas,
   ein Reglerventil (24) zur gesteuerten Abgabe des Gases aus dem Minigastank (22) an
   einen Brenner, z. B. einen Vormischbrenner (25) zum Verbrennen des brennbaren Gases vorzugsweise im Wesentlichen mit Luftüberschuss und
   einen Stofftauscher (26) zur Erwärmung der Luft durch die mittels des Vormischbrenners (25) erzeugte Hitze,
   wobei das Mundstück (3) mit dem Heizstab (2), in Form und Abmessungen einer Zigarette oder Zigarre ähnlich, zur Abgabe eines inhalierbaren Aerosols, lösbar verbunden ist und die Steuerung des Brenners (25) durch das Reglerventil (24) mittels des durch den Sog eines Benutzers am Mundstück (3) erzeugten Unterdrucks und/oder Luftstroms erfolgt.

Dabei werden die Abgase des Brenners (25), teilweise oder vollständig, mit Umgebungsluft vermischt durch das Mundstück (3) geführt, um durch die so erzeugte Heißluft die im Wirk- und/oder Aromastoffdepot (32) befindlichen Substanzen zu verdampfen, z. B. um sie als Aerosol inhalieren zu können.

Die Vorteile eines solchen Verfahrens und einer darauf basierenden Vorrichtung sind vielfältig. Es erfolgt erstens keine Rauchbelästigung durch Verbrennung komplexer Stoffe, wie Tabak, und zweitens entsteht wegen der extrem sauberen Verbrennung eines brennbaren Gases kein so genannter Nebenstromrauch, wodurch Dritte nicht durch Passivrauchen geschädigt werden. Drittens, dadurch dass im Gegensatz zu einer herkömmlichen Zigarette die Wirk- und/oder Aromastoffe und deren Anzahl gezielt ausgewählt werden können, ist deren Inhalation gesundheitlich bei weitem unbedenklicher als bei einer normalen Zigarette und so kann eine krebserregende (karzinogene) Wirkung gezielt ausgeschlossen werden. Anders ausgedrückt, da keine Verbrennung im herkömmlichen Sinne erfolgt, wird eine kontrollierte Aerosolzusammensetzung ermöglicht, ohne Dritte zu belästigen.

Dieser für die Akzeptanz vom Benutzer entscheidende Vorteil wird durch die Verflüchtigung der Wirk- und/oder Aromastoffe (Bildung eines inhalierbaren Aerosols) ausschließlich durch die erwärmte Luft aus dem Stofftauscher erreicht, d.h. ohne das Depot durch direkten Kontakt mit der Energiequelle zu erhitzen oder gar zu verbrennen. Dabei ist ein inhalierbares Aerosol im Sinne dieser Erfindung im Prinzip ein Gemisch aus festen und/oder flüssigen Schwebeteilchen und Luft. Bevorzugt handelt es sich beim inhalierbaren Aerosol um einen Nebel, d.h. ein Gemisch bestehend hauptsächlich aus flüssigen Schwebeteilchen und Luft, vorzugsweise weitestgehend frei von Feinstaub.

Im Gegensatz zu bekannten Verfahren, die auf einer elektrischen Energiequelle zur Aerosolbildung basieren, benötigt das erfindungsgemäße Verfahren und die darauf basierende Vorrichtung keinen Hochleistungsakku, was niedrigere Herstellungskosten ermöglicht und erheblich weniger Entsorgungsprobleme bereitet.

Weiterhin stellt brennbares Gas, d. h. z. B. so genanntes Feuerzeuggas, vorzugsweise Propan- und/oder Butangas, als Energiequelle einen weiteren Vorzug der Erfindung dar, da hiermit gute Heizwerte und hervorragende Umweltverträglichkeit kombinierbar sind. Außerdem erlaubt die Wahl dieser Energiequelle auf bereits existierende Infrastrukturen und Erfahrungen zurückzugreifen, was die Herstellungskosten erheblich senkt.

Darüber hinaus ermöglicht die Form und die Abmessungen der kombinierten Vorrichtungsteile Heizstab (2) und Mundstück (3) eine Benutzung die der einer herkömmlichen Zigarette sehr ähnlich ist. In diesem Zusammenhang, bedeutet der Ausdruck "Form und Abmessungen einer Zigarette oder Zigarre", dass die kombinierten Vorrichtungsteile Heizstab (2) und Mundstück (3) eine grundsätzlich zylindrische Form haben und einen Durchmesser zwischen ca. 6 bis ca. 14 mm, bevorzugt zwischen ca. 8 und 11 mm und eine Länge von ca. 50 bis ca. 150 mm, bevorzugt zwischen ca. 75 und ca. 105 mm aufweisen. Die einfache Handhabung durch den Benutzer wird außerdem durch ein im Vergleich zu bekannten Vorrichtungen sehr geringes Gewicht unterstützt, sodass die Vorrichtung auch im Mund problemlos tragbar ist ohne dass sie permanent in der Hand gehalten werden muss.

Die für die Imitation des Rauchaktes herkömmlicher Zigaretten bzw. (kleiner) Zigarren unabdingbaren geringen Abmessungen des Heizstabs (2) sind alleine dadurch möglich, dass der Minigastank (22) im Heizstab (2) in seinem Volumen auf eine Konsumeinheit begrenzt und auf eine Zündfunktion für den Vormischbrenner (25) im Heizstab (2) verzichtet wird. Die im Sinne der Aufgabe der Erfindung erforderliche Wiederverwendbarkeit des Heizstabs (2) wird dadurch gewährleistet, dass eine Tankstation (1) mit den Funktionen Wiederbefüllen des Heizstabs (2) mit Flüssiggas und Entzünden des Heizstabs (2) die Vorrichtung komplettiert.

Die Gemeinsamkeiten zur Handhabung einer Zigarette werden noch zusätzlich dadurch verstärkt, dass das Anzünden (d.h. das Starten der Verbrennung, bzw. Beginn des Rauchakts) der erfindungsgemäßen Vorrichtung durch einen Sog durch den Benutzer an der Vorrichtung und gleichzeitiges Halten der dem Mundstück abgewandten Seite an eine Flamme, die im Normalfall von der Tankstation zur Verfügung gestellt wird (alternativ kann jedoch auch z. B. ein übliches Feuerzeug oder auch Zündholz verwendet werden), erfolgt.

Im Gegensatz zu bekannten Produkten ist also kein manueller Schalter notwendig um die Energiequelle zu starten und durch die Wahl von brennbarem Gas als Energiequelle ist wegen dessen hohem Heizwert eine Gastankfüllung ausreichend für die Konsumdauer einer herkömmlichen Zigarette. Nach Verbrauch einer Tankfüllung erlischt der Brenner von selbst, was verhindert, dass der Benutzer vergisst die Vorrichtung auszuschalten und so der Sicherheit dient. Vor jedem weiteren Konsum, muss der Heizstab wieder in der Tankstation befüllt werden.

Diese dem Anzünden einer Zigarette sehr ähnliche Prozedur wird durch ein Reglerventil (24) ermöglicht, das erst ausreichend Gas zum-Entzünden an den Vormischbrenner (25) freigibt, sobald der Benutzer am Mundstück (2) der Vorrichtung zieht.

Das Reglerventil (24) ist steuerbar mittels eines durch den Sog eines Benutzers erzeugten Unterdrucks und/oder Luftstroms. Dabei umfasst das Ventil einen Regelkörper, sowie Mittel zur Erzeugung einer Rückstellkraft (241), wobei der Regelkörper einen ersten minimal offenen und einen zweiten offenen Zustand aufweist, wobei der Regelkörper mittels des erzeugten Unterdrucks und/oder Luftstroms aus dem ersten minimal offenen Zustand entgegen einer Rückstellkraft in den zweiten offenen Zustand versetzbar ist und die Mittel zur Erzeugung einer Rückstellkraft derart gewählt sind, dass sie den Regelkörper in den ersten minimal offenen Zustand zurücksetzen können.

Der Vorteil eines solchen Ventils ist, dass es möglich ist, z. B. im Fall der Reglung eines brennbaren Gases, mindestens zwei grundsätzliche Betriebsarten darzustellen: Betriebsflamme (offen) und Pilotflamme (minimal offen).

Darüber hinaus, sobald der Benutzer einen genügenden Unterdruck und/oder Luftstrom innerhalb der Vorrichtung erzeugt, wird der Regelkörper dadurch in einen offenen Zustand gebracht und das Gas kann entweichen. Wird das Gas gleichzeitig durch den Benutzer entzündet verbrennt es im Brenner.

Eine Reglung per Luftstrom wird im Prinzip dadurch erreicht, dass am Regelkörper Strömungshindernisse angeordnet sind die sich innerhalb des Luftkanals befinden und so mittels des durch den Sog erzeugten Luftstroms betätigt werden. Eine Reglung per Unterdruck wird dann erreicht, wenn die durch den Sog entstandene Druckdifferenz vor und hinter dem Ventilkörper dazu genutzt wird diesen zu verstellen. Beide Möglichkeiten zur Reglung sind natürlich kombinierbar. Als genügender Luftstrom kann z. B. ein Volumenstrom zwischen 15 und 20 ml/s, typischerweise ca. 17,5 ml/s gelten. Ein entsprechender Unterdruck beträgt z. B. zwischen 50 und 500 mbar, bevorzugt zwischen 100 und 200 mbar.

Damit eine Reglung des Durchflusses erfolgen kann, wird durch den Sog beim Öffnen des Ventils ein Mittel zur Erzeugung einer Rückstellkraft (241), z. B. eine Feder, gespannt.

Hört der Benutzer auf am Mundstück zu ziehen, wird der Regelkörper durch diese Rückstellkraft zurückgedrückt.

So ist in diesem Zusammenhang unter "minimal offen" oder den entsprechenden Ausdrücken "minimaler Durchfluss" oder "minimaler Strömungsquerschnitt" ein Zustand zu verstehen in dem eine geringe Menge an Gas durchströmt, die gerade ausreicht damit die Flamme in einem nachgeschalteten Brenner nicht erlischt. Der daraus resultierende Betriebszustand, auch Pilotflamme genannt, ermöglicht es dem Benutzer mehrere Züge zu tätigen ohne jedes Mal den Brenner neu zu zünden, dabei aber möglichst wenig Gas zu verbrauchen, was einer kompakteren Bauweise wie im Zusammenhang mit der erfindungsgemäßen Vorrichtung zugute kommt.

Bevorzugt, gibt der Regelkörper in dem zweiten offenen Zustand einen Strömungsquerschnitt frei der abhängig vom Unterdruck und/oder Luftstrom variabel zwischen einem minimal offenen bis ganz offenen Zustand steuerbar ist, womit erreicht wird, dass eine feinere Reglung abhängig von der Sogkraft des Benutzers, bzw. dessen Rauchgewohnheiten möglich ist.

In einer weiteren Ausgestaltung dieser Vorrichtung, umfasst diese zusätzlich ein Hauptventil (23) zwischen Minigastank (22) und Reglerventil (24) welches durch Verbinden des Mundstücks (3) mit dem Heizstab (2) geöffnet und durch Ablösen des Mundstücks (3) wieder geschlossen wird. Damit wird gewährleistet, dass kein Gas entweichen kann, solange die Vorrichtung nicht betriebsbereit ist, d.h. solange das Mundstück nicht auf den Heizstab aufgesetzt ist.

In einer weiteren Ausgestaltung kann der Regler (24) einen weiteren, dritten geschlossenen Zustand nach Trennung von Heizstab (2) und Mundstück (3) einnehmen, der bei erneutem Aufstecken eines Mundstücks beibehalten wird, bis ein Unterdruck und/oder Luftstrom am Regelkörper anliegt, der diesen, bei Überschreiten eines Mindestwertes, je nach Stärke, in den minimal offenen, den offenen oder einen Zustand zwischen minimal offen und offen versetzt.

Durch diesen dritten geschlossenen Zustand ist es möglich, einen mit einem neuen Mundstück (3) versehenen und betankten Heizstab (2) bis zum gewünschten Beginn des Rauchaktes, d. h. bis zum gewünschten Zeitpunkt des Entzündens des Heizstabs, aufzubewahren oder z. B. in der Hand zu halten, ohne dass Gas entweicht.

Der Vormischbrenner (25) des Heizstabs (2) ist vorzugsweise ein Flüssiggas-Vormischbrenner, d.h. das brennbare Gemisch wird bereits vor dem Düsenaustritt gebildet, sodass eine vollständige Verbrennung des Flüssiggases gewährleistet wird. Zu dieser Art von Brennern werden z. B. Nainen-, Ikari- ,Matrix- oder Katalyt-Brenner gezählt und sind teilweise aus Sturmfeuerzeugen und kleinen Handgaslötgeräten bekannt.

Die Ähnlichkeit zu einer herkömmlichen Zigarette während der Benutzung kann weiterhin vervollständigt werden indem ein Glühen oder Glimmen an der dem Mundstück abgewandten Seite auftritt sobald der Benutzer am Mundstück zieht. Dies wird z. B. erreicht mit einem Metallgitter das in der Flamme oder der Flammfront angeordnet ist. Bei jedem Zug an der Vorrichtung wird so durch die größere und wärmere Betriebsflamme das Gitter zum Glühen gebracht. Das Gitter sollte dabei hochhitzebeständig sein, d.h. nicht bei den in der Flamme erreichten Temperaturen verglühen, beispielsweise kann ein solches Gitter aus Wolfram oder einer Wolfram-Legierung bestehen.

In einer bevorzugten Ausgestaltung des Brenners (25) als Katalytbrenner ist das Glühgitter ggf. gleichzeitig Träger für einen Katalysator, wie z. B. Platin oder Palladium.

Da zum Teil sehr hohe Temperaturen im Betrieb erreicht werden können, kann es sinnvoll sein einen Überhitzungsschutz, z. B. einen Bimetallregler vorzusehen. Dieser kann so gestaltet werden, dass er durch Ausdehnung bei Überhitzung das Reglerventil schließt und so die Gaszufuhr zum Brenner unterbricht. Dies kann z. B. erfolgen indem er durch seine Ausdehnung dem Reglerventil einen zusätzlichen (vierten) Zustand erlaubt in dem das Reglerventil geschlossen ist oder indem er das Reglerventil in den dritten Zustand zurückschiebt. Beides kann dadurch erreicht werden, dass die Mittel zur Erzeugung einer Rückstellkraft (241), z. B. eine Feder, so beeinflusst werden, z. B. durch Verschieben des Federwegs, dass sie das Ventil in eine vierte geschlossene oder, alternativ, in seine dritte geschlossene Position zurücksetzen können.

Die Erwärmung der zur Verflüchtigung der im Depot enthaltenen Substanzen benötigten Luft erfolgt erfindungsgemäß durch einen so genannten Stofftauscher (26). Da eine Temperaturerhöhung der Inhalationsluft z. B. um mindestens 180° C erforderlich ist, damit die Inhalationsluft beim Verlassen des Depots noch eine Temperatur von 100° C besitzt, beinhaltet der Heizstab (2) bevorzugt einen Stofftauscher (26) in dem die Abgase der Verbrennung des brennbaren Gases mindestens zum Teil als Warmluft genutzt werden, wobei zur Einstellung der benötigten Temperatur Frischluft hinzu gemischt werden kann. So können z. B. 3 bis 100 %, vorzugsweise 5 bis 50 % der Abgase in den Warmluftstrom eingeführt werden.

Wegen der kompakten Abmessungen einer erfindungsgemäßen Vorrichtung, ist diese bevorzugt so gestaltet, dass ein oder mehrere zu dem oder den Warmluftausgängen führende Warmluftkanäle (27) koaxial um den Gastank (21) des Heizstabs (2) angeordnet sind. Diese Warmluftkanäle (27) führen die erwärmte Luft vom Stofftauscher (25) zu den Warmluftausgängen, wo sie bei aufgesetztem Mundstück durch die Kammer oder Kammern des Depots (32) zwecks Verflüchtigung der darin vorhandenen Wirk- und Aromastoffe zum Mund des Benutzers geführt wird.

Bevorzugt erfolgen beim Genuss eine Anregung der Geschmacksnerven und eine Stimulation der Nikotinrezeptoren. Daher enthält die Vorrichtung in einer bevorzugten Ausführungsform in dem Wirk- und/oder Aromastoff-Depot (32) als Wirkstoff Nikotin und ggf. Aromastoffe, sowie ggf. weitere Zusatz- und Hilfsstoffe, wie Reizstoffe, z. B. Capsaicin, Stabilisatoren z. B. Propylenglycol, Verflüchtigungsvermittler, z. B. Ethanol, usw.

Bei einem Depot (32) mit mehreren Inhaltsstoffen kann es, z. B. aufgrund der unterschiedlichen benötigten Verdampfungsenergien, vorteilhaft oder notwendig sein, dass der oder die Wirkstoff(e), ggf. Aromastoff(e), sowie ggf. weitere Zusatz- und Hilfsstoffe in mehreren Kammern einzeln und/oder gemischt vorliegen. Dabei können die Kammem unterschiedliche Eigenschaften haben in Bezug auf Abmessungen und Form um die gewünschte Dosierung der einzelnen Komponenten zu erreichen, z. B. durch Variation des Strömungsquerschnitts und/oder der Depotmaterialien, usw.

Da die oben genannten Gase, wie Butan- und/oder Propan, bei stöchiometrischer Verbrennung, d.h. mit einem λ_{Luft}-Wert (Luftverhältnis) ≥ 1 vollständig zu Kohlenstoffdioxid (CO₂) und Wasser (H₂O) reagieren, ist das Verbrennungsabgas aus gesundheitlicher Sicht sauber. Trotzdem kann es auch bei einer im Wesentlichen mit Luftüberschuss arbeitenden Verbrennung vorkommen, speziell bei Lastwechselreaktionen, dass dieser λ_{Luft}-Wert zeitweise kleiner als 1 ist, d.h. die Verbrennung ist teilweise unvollständig, was in der Praxis im Fall eines Stofftauschers, d.h. wenn ein Teil der Abgase direkt zur Erwärmung durch Vermischen mit Umgebungsluft benutzt werden, geringe Mengen an Kohlenstoffmonoxid (CO) in der Atemluft bedeuten würde.

Deshalb sieht eine weitere Ausführungsform der Vorrichtung vor, dass ein Kohlenstoffmonoxid-Oxydationskatalysator, z. B. ein Hopkalit-Katalysator, dem Stofftauscher (26) nachgeschaltet ist, wodurch etwaige geringe Mengen an Kohlenstoffmonoxid in unbedenkliches Kohlenstoffdioxid (CO₂) umgewandelt werden.

Ein weiterer Aspekt der Erfindung ist es eine Tankstation (1) zum wiederabnehmbaren Verbinden und Befüllen des Heizstabs (2) einer erfindungsgemäßen Vorrichtung bereitzustellen, welche einen Gasvorratstank (11), dessen Volumen das Mehrfache des Volumens des Minigastanks (22) des Heizstabs (2), z. B. mindestens das 100-fache, vorzugsweise mindestens das 200-fache Volumen, beträgt und ein form- und/oder kraftschlüssig und gasdicht mit dem Füllventil (21) des Heizstabs verbindbares Auslassventil (17) beinhaltet. Diese ermöglicht es dem Benutzer den wieder verwendbaren Heizstab (2) nach Abnahme (bzw. vor Aufsetzen) des Mundstücks (3) mit brennbarem Gas zu füllen. Ohne eine Tankstation (1) kann die Aufgabe der Erfindung, die unter anderem beinhaltet, dass die Handhabung und die Kosten der erfindungsgemäßen Vorrichtung keinen großen Unterschied zum Konsum herkömmlicher Zigaretten bzw. (kleiner) Zigarren aufweisen darf, nicht vollständig erfüllt werden.

Bevorzugt dient die Tankstation (1) nicht nur dazu den Heizstab (2) aufzufüllen, sondern auch um ihn bei Nicht-Benutzung aufzubewahren. Deshalb umfasst die Tankstation (1) in einer weiteren Gestaltungsform weiterhin Mittel zur Aufbewahrung eines Heizstabs mit einer das Füllventil (17) zu mindest teilweise umschließende Aufbewahrungstasche (18) zur Aufnahme eines mundstückseitigen Teils des Heizstabs (2), sowie einem umsetzbaren Deckel (19) der in einer geschlossenen Stellung die entgegengesetzte Seite des Heizstabs (2) überdeckt. Da dieser Deckel (19) die ggf. noch heiße Spitze des Heizstabs (2) überdeckt, ist er bevorzugt als Hitzeschild ausgestaltet, z. B. aus einem hitzebeständigen und vorzugsweise isolierenden Material, damit der Benutzer die Tankstation (1) mit eingelegtem Heizstab (2) sofort gefahrlos z. B. in die Tasche stecken kann.

In einer weiteren Ausgestaltung enthält die Tankstation (1) zusätzlich eine Zündvorrichtung (163), z. B. einen Piezo-Zünder, mit der sich die Vorrichtung aus Heizstab (2) und Mundstück (3) anzünden lässt.

In einer weiteren bevorzugten Ausgestaltung umfasst die Tankstation (1) zusätzlich zu einer solchen Zündvorrichtung (163) weiterhin ein Brennerventil (162) und einen Brenner (164) z. B. einen Diffusionsbrenner, einen Teilvormischbrenner oder bevorzugt einen Vormischbrenner, wobei das Brennerventil (162), die Zündvorrichtung (163) und der Brenner (164) in einem solchen Fall ein komplettes Feuerzeug darstellen, welches primär zum Entzünden der Vorrichtung (Heizstab) dient und sekundär, unabhängig vom Heizstab (2), wie ein herkömmliches Feuerzeug benutzt werden kann.

Der Minigastank (22) des Heizstabs muss aufgrund des knappen zur Verfügung stehenden Bauraumes vor jedem Konsum vollständig mit Flüssiggas, welches zu mindestens 90%, vorzugsweise zu 100% in flüssiger Phase vorliegen muss, befüllt werden, um den Brenner (25) über die geforderte Konsumdauer betreiben zu können. Die Tankstation (1) sollte, vorzugsweise aus Gründen der erforderlichen einfachen Handhabung, den Heizstab (2) ohne Anleitung des Benutzers nach dem Einlegen in die Aufbewahrungstasche (18) mit Flüssiggas befüllen.

Um dies zu erreichen muss bei verbundenen und geöffneten Nachfüllventilen (17, 21) von Tankstation (1) und Heizstab (2) auf Tankstationsseite bis zur nahezu vollständigen Entleerung des Gasvorratstanks (11) der Tankstation (1) stets ein Druck anliegen, der über dem zur Verflüssigung des Flüssiggases notwendigen Dampfdruck liegt. So ist garantiert, dass unabhängig von der räumlichen Position der Tankstation (1) eine Befüllung des Heizstabs (2) mit flüssigem Gas gewährleistet ist.

Dies wird gelöst durch eine Tankstation (1) mit einem Gasvorratstank (11), der durch einen entlang einem Verstellweg axial beweglichen Kolben (12) in eine mit einem inerten Gas, vorzugsweise Stickstoff, gefüllte erste Kammer (13), und eine mit brennbarem Gas, vorzugsweise Propan- oder Butangas, gefüllte zweite Kammer (14) unterteilt ist, wobei der Druck in der ersten mit inertem Gas gefüllte Kammer (13) über den gesamten Verstellweg des Kolbens (12) ausreicht, um das Gas in der mit brennbarem Gas gefüllten Kammer (14) in flüssiger Phase zu halten.

Wenn die Flüssiggaskammer (14) leer ist bedeutet dies, dass die Druckgaskammer (13) den vollen Raum im Gasvorratstank (11) einnimmt und der Druck im Druckgas z. B. 3 bar beträgt, wenn für die Flüssiggaskammer (14) z. B. Butan vorgesehen ist. Füllt man die Flüssiggaskammer (14) mit Butan, liegt das Butan so stets in flüssiger Phase vor und das Gas in der Druckgaskammer (13) wird über den Kolben (12) komprimiert. Nimmt, bei gegebenem Beispiel, nun die Flüssiggaskammer (14) nach dem Befüllen 85% des Gasvorratstankvolumens ein, liegt der Gesamtdruck des Systems bei ca. 21 bar.

Um den Gasvorratstank (11) vor unzulässigen Drücken, wie sie z. B. bei direkter Sonneneinstrahlung in Fahrzeugen auftreten können, zu schützen, ist die mit inertem Gas gefüllte Kammer (13) über ein Überdruckventil (131) mit der Umgebung verbunden. Da somit bei Überdruck kein brennbares Flüssiggas abgelassen wird, besteht keine Explosionsgefahr.

Die Flüssiggaskammer (14) ist über einen Druckminderer (15) mit dem Auslassventil (17) und in einer Ausgestaltungsvariante der Tankstation (1) mit einem integrierten Feuerzeug (16), eventuell mit einer zwischengeschalteten Drossel (161), mit dem Brennerventil (162) verbunden.

Der Druckminderer (15) ist so ausgelegt, dass der Hinterdruck ausreicht, um das Flüssiggas im dahinter liegenden System in der flüssigen Phase zu halten. So ist bei jedem Betanken des Heizstabs (2) gewährleistet, dass dieser zum einen stets vollständig mit flüssigem Gas befüllt wird und zum anderen weder der Heizstab (2) noch das Brennerventil (162) mit einem zu hohen Druck, wie er im Gasvorratstank (11) bei nicht vollständiger Entleerung der Flüssiggaskammer (14) vorliegt, zu schützen.

Obwohl die Tankstation (1) als Einwegprodukt ausgestaltet werden kann, ist es vorteilhaft wenn sie weiterhin ein Füllventil (141) zum Befüllen des Gasvorratstanks (11) umfasst, das es erlaubt sie nach Entleerung des Vorratstanks wieder zu befüllen und weiter zu verwenden.

Die Erfindung betrifft selbstverständlich auch die einzelnen Komponenten, d. h. den wieder verwendbaren Heizstab (2), das Einweg-Mundstück (3), sowie die dazu passende Tankstation (1). Bevorzugt betrifft die Erfindung auch ein Kit umfassend eine Tankstation und einen Heizstab wie oben beschrieben, gegebenenfalls mit einer Vielzahl an Mundstücken.

Nur unter Verwendung aller drei Komponenten ist eine Vorrichtung darstellbar, die die Aufgabe der Erfindung, eine möglichst vollständige Imitation des Rauchaktes bereitzustellen, erfüllt.

Folgende vorteilhafte Eigenschaften können durch die vorgestellte Vorrichtung realisiert werden:
➢ Erscheinungsbild einer Zigarette (Heizstab mit Mundstück)
➢ Nikotin-Emission im Hauptstrom, wie bei einer Zigarette
➢ Geschmackserlebnis, wie beim Rauchen einer Zigarette
➢ Leichtes Kratzen bzw. Brennen im Mund- und Rachenraum, wie beim Rauchen einer Zigarette
➢ Handhabung ähnlich der einer Zigarette
➢ Geringe Herstellkosten, dadurch geringer Verkaufspreis von Tankstation und Heizstab, vorzugsweise VK < 10 €
➢ Mindestens 50 %, vorzugsweise mindestens 95 % Schadstoffreduktion im Nebenstrom gegenüber einer herkömmlichen Zigarette (Abgas: nur Luft + Wasser und CO₂)
➢ Mindestens 20%, vorzugsweise mindestens 50 %, je nach Auswahl der Inhaltsstoffe sogar mindestens 90 % Schadstoffreduktion im Hauptstrom gegenüber einer herkömmlichen Zigarette (Abgas (+Luft) + z. B. Nikotin und Aromen)
➢ Eine gezielte Ausschließung von eventuell gesundheitsschädigenden Substanzen, z. B. Kanzerogene, sowie die Möglichkeit der gezielten Dosierung der Inhaltsstoffe und Emissionen unterhalb der zulässigen Grenzwerte.

### Kurze Beschreibung der Figuren

Im Folgenden werden nun Ausgestaltungen der Erfindung anhand der beiliegenden Figuren beschrieben.
Fig. 1 zeigt eine schematische Darstellung des Verfahrens in Form eines Flussdiagramms. Die Parallelogramme stellen die am Verfahren beteiligten Stoffe, die Rechtecke die Teilprozesse des Verfahrens dar. Die dünnen Pfeile symbolisieren die durch die Kinetik des Flüssiggases verursachte Strömung, die dicken Pfeile den durch den Sog verursachten Luftstrom. Die gestrichelte Linie stellt den Einfluss des Unterdrucks und/oder Luftstroms auf den Mengenstrom des Flüssiggases dar.
Fig.2 beinhaltet schematische Darstellungen der Vorrichtungskomponenten Tankstation 1, Heizstab 2 und Mundstück 3.
   a) zeigt den in der Tankstation 1 eingelegten Heizstab 2. Die Nachfüllventile 17 und 21 sind geöffnet, wodurch der Heizstab 2 betankt wird.
   b) zeigt, wie ein Mundstück 3 auf den Heizstab 2 durch Verbindung der Bajonettverschlussteile 27 und 31 aufgeschraubt wird, wodurch das Hauptventil 23 des Heizstabs 2 geöffnet wird.

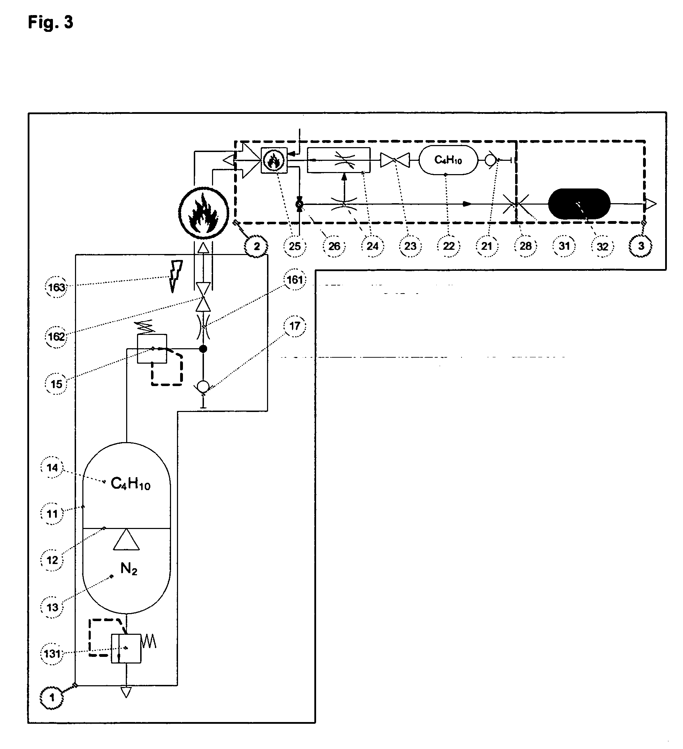

Fig. 3 ist eine schematische Darstellung der Vorrichtungskomponenten und zeigt, wie die miteinander verschraubten Komponenten Heizstab 2 und Mundstück 3 mit Hilfe der Tankstation 1 entzündet werden.

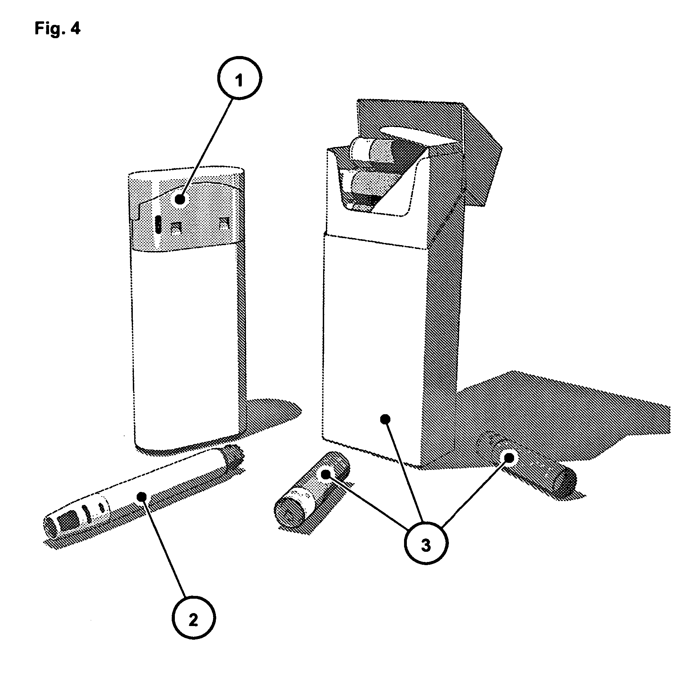

Fig. 4 zeigt eine perspektivische Ansicht der drei zur Vorrichtung gehörenden Komponenten, Tankstation 1, Heizstab 2 und Mundstück 3, sowie eine Schachtel mit Mundstücken 3.

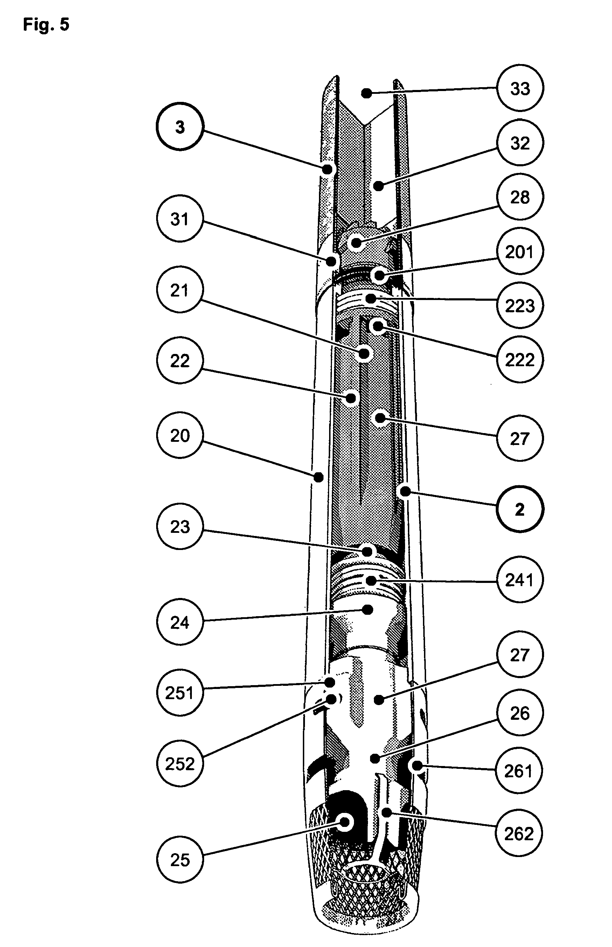

Fig. 5 zeigt eine perspektivische Schnittansicht des Heizstabs 2 mit aufgeschraubtem Mundstück 3.

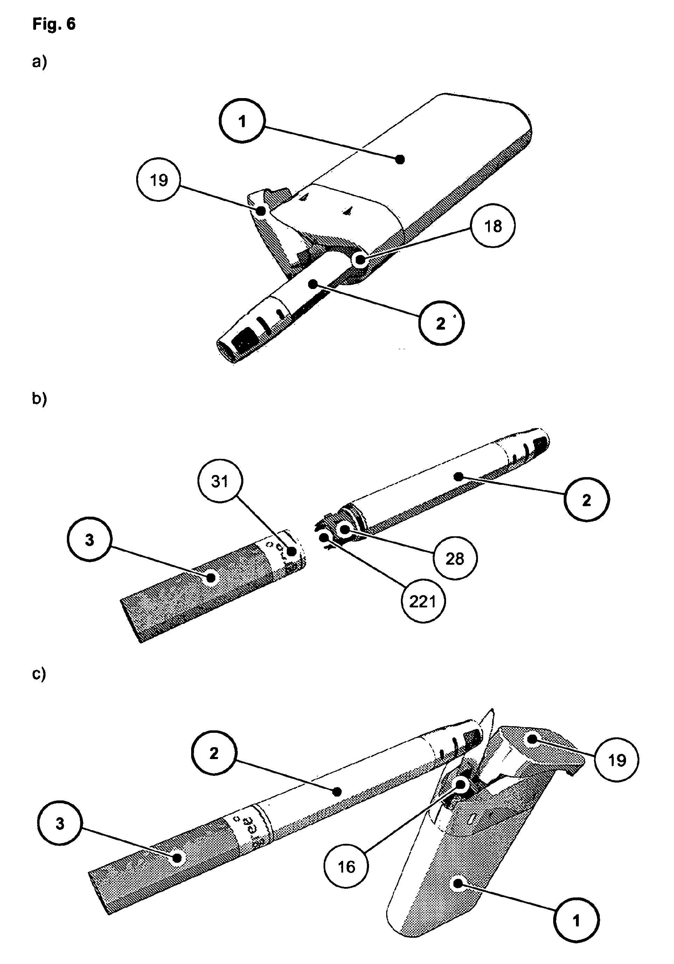

Fig. 6 zeigt eine perspektivische Ansicht einer Ausführungsform a) einer Tankstation 1 mit geöffnetem Deckel 19 und Heizstab 2 der aus der Tankstation 1 entnommen wird, b) ein Mundstück 3 vor dem Aufschrauben auf den Heizstab 2 und c) ein Heizstab 2 mit aufgestecktem Mundstück 3 während des Zündvorgangs durch die Tankstation 1.

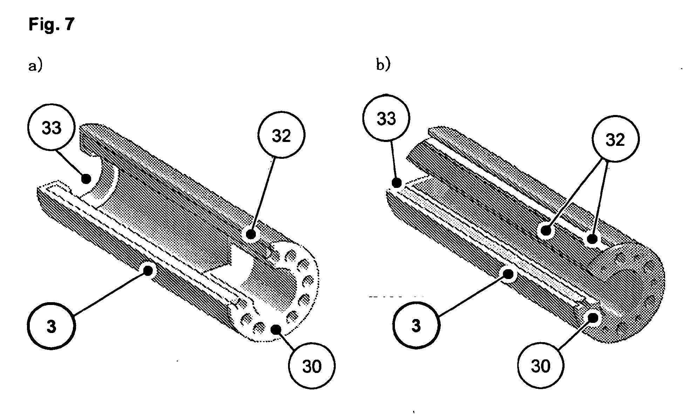

Fig. 7 zeigt a) ein Mundstück 3 mit Heißlufteintrittsöffnungen 30, einem Ein-Kammer-Depot 32 und Aerosolaustrittsöffnungen 33, sowie b) ein Mundstück 3 mit einem Mehr-Kammer-Depot 32.

Weitere Einzelheiten und Vorteile der Erfindung können der nachfolgenden ausführlichen Beschreibung möglicher Ausführungsformen der Erfindung anhand der beiliegenden Figuren entnommen werden.

### Beschreibung mehrerer Ausgestaltungen der Erfindung

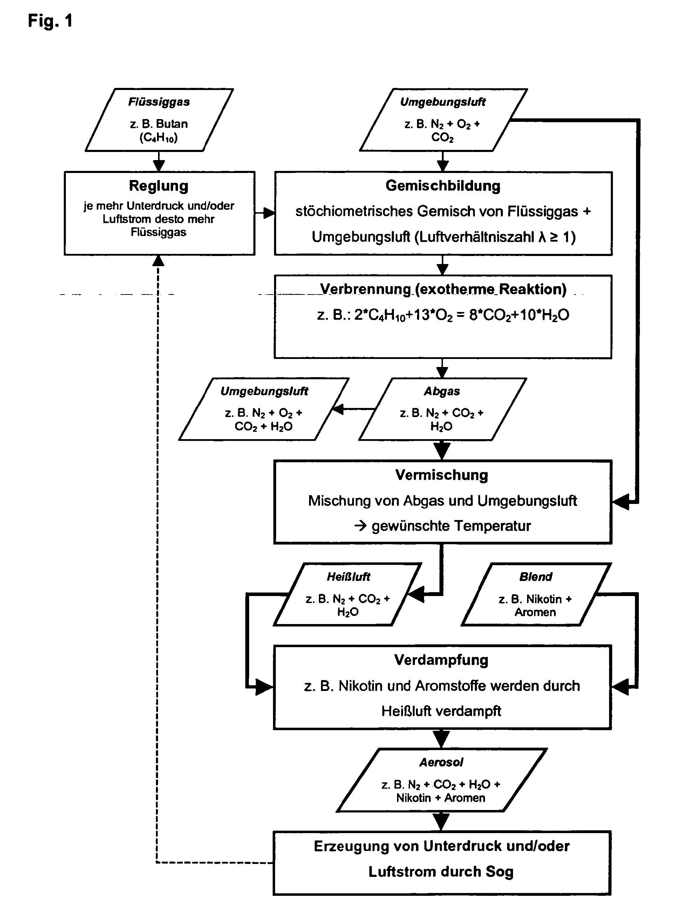

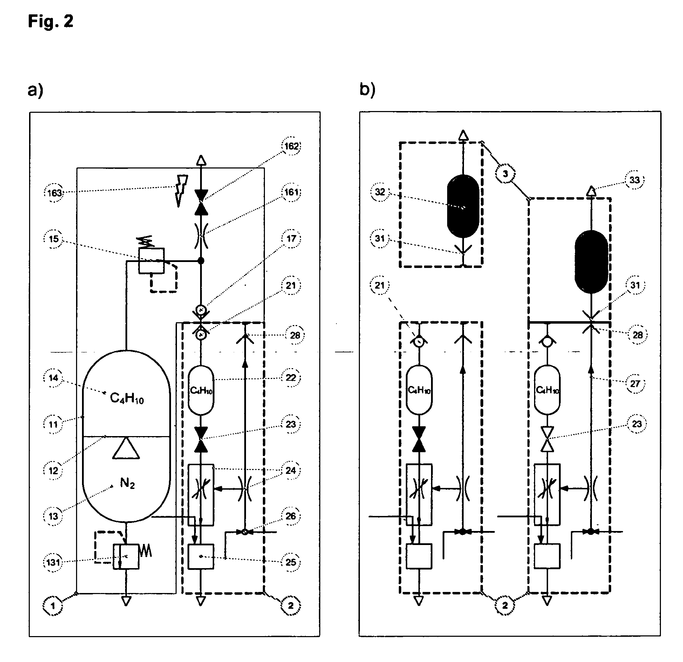

### 1. Ausgestaltungen der Tankstation 1 (s. Fig. 1 und 2)

Die Tankstation 1 dient prinzipiell zum Nachtanken, ggf. auch zum Aufbewahren und Entzünden des Heizstabs 2. Mit dem (eingelegten) Heizstab bildet sie die transportable Hardware des Produktes. In einer bevorzugten Ausgestaltung ist sie ein erweitertes Feuerzeug, welches den erfindungsgemäßen Heizstab 2 in sich aufnehmen und betanken kann.

### 1.1 Ausgestaltung T1 einer Tankstation

In einer ersten Ausgestaltung dient die Tankstation 1 ausschließlich dem Nachtanken des Heizstabs 2 und besteht deshalb nur aus einem Gasvorratstank 11 mit Nachfüllventil 141 zum Wiederbefüllen der Tankstation 1 und einem Auslassventil 17 zum Nachtanken des Heizstabs 2. Bevorzugt kann der Heizstab 2 zur Aufbewahrung bei Nichtgebrauch in der Tankstation 1 verbleiben. Zum Zünden des Heizstabs 2 muss entweder ein Zünder im Heizstab untergebracht sein (erhöhter Platzbedarf im Heizstab) oder ein zusätzliches Feuerzeug verwendet werden.

### 1.2 Ausgestaltung T2 einer Tankstation mit Zünder

Wie Ausgestaltung T1, jedoch mit zusätzlich integriertem Zündmechanismus 163 zum Zünden des Heizstabs 2. Gegenüber Ausgestaltung T1 entfällt jedoch die Notwendigkeit eines zusätzlichen Feuerzeugs (kein erhöhter Platzbedarf im Heizstab oder in der Tasche des Konsumenten).

### 1.3 Ausgestaltung T3 einer Tankstation mit Feuerzeug

Wie Ausgestaltung T1, jedoch mit zusätzlich integriertem Feuerzeug 16 zum Zünden des Heizstabs 2. So kann die Tankstation 1 auch als herkömmliches Feuerzeug genutzt werden, was vor allem Kunden zugute kommt, die neben der rauchfreien Zigarette auch weiterhin herkömmliche Zigaretten bzw. kleine Zigarren konsumieren möchten.

### 1.3.1 Tankstationsausgestaltung T3 im Detail (s. Fig. 1 und 2)

### 1.3.1.1 Gasvorratstank

Der Gasvorratstank 11 ist das vom Bauraum her größte Bauteil der Tankstation 1 und die Energiequelle des Gesamtsystems rauchfreie Zigarette. Der mit handelsüblichem Feuerzeuggas wieder befüllbare Flüssiggastank 11 versorgt
➢ das in der Tankstation 1 integrierte Feuerzeug 16,
➢ den Heizstab 2 in der Heizstabaufnahme 18, wenn der Multifunktionsdeckel 19 bei eingelegtem Heizstab 2 gedrückt wird bzw. geschlossen ist.

Der Gasvorratstank 11 ist durch einen axial beweglichen, gasdichten Kolben 12 in eine Stickstoffkammer 13 und eine Butankammer 14 unterteilt. Ist die Butankammer 14 leer, so nehmen die Stickstoffkammer 13 und der Kolben 12 den gesamten Bauraum innerhalb des Gasvorratstanks 11 ein. In diesem Zustand liegt der Druck in der Stickstoffkammer 13 nur knapp über dem Dampfdruck von Butan, d.h. über dem notwendigen Druck, um Butan bei Raumtemperatur zu verflüssigen. Wird die Butankammer 14 gefüllt, so bewegt das durch den in der Stickstoffkammer 13 herrschenden Druck flüssige und inkompressible Butan den Kolben 12, wodurch die Butankammer 14 größer und die Stickstoffkammer 13 kleiner wird. Der Druck in beiden Kammern steigt durch die Kompression des Stickstoffs und liegt dadurch in jedem Füllzustand der Butankammer 14 über dem Dampfdruck von Butan, wodurch die Butankammer 14 stets zu 100% mit flüssigem Butan gefüllt ist.

Um den Gasvorratstank 11 gegen Drücke abzusichern, die eine Zerstörung des Tanks zur Folge hätten ist die Stickstoffkammer 13 über ein Überdruckventil 131 mit der Umgebung verbunden. Steigt der Druck im Gasvorratstank 11 über einen zulässigen Wert, so öffnet das Ventil und lässt Stickstoff in die Umgebung ab, bis der Druck im Flüssiggastank 11 wieder unterhalb des zulässigen Höchstwertes liegt. Durch diese Anordnung wird zum einen verhindert, dass der Tank explodiert und eventuell Menschen zu Schaden kommen und zum anderen, dass Butan ungewollt entweichen kann.

Die Butankammer 14 ist über einen Druckminderer 15 mit dem integrierten Feuerzeug 16 und dem Heizstabfüllventil 17 verbunden. Der Druckminderer 15 ist so ausgelegt, dass der Heizstab 1 stets mit einem Druck befüllt wird, der das Butan in der flüssigen Phase hält, wodurch eine maximale Füllung des Minigastanks 22 im Heizstab 2 gewährleistet ist. Der Hinterdruck des Druckminderers 15 liegt knapp über dem Dampfdruck von Butan und schützt das integrierte Feuerzeug 16 und das Auslassventil 17 vor für diese Bauteile zu hohen Drücken, wie sie innerhalb des Gasvorratstanks 11 fast über den gesamten Lebenszyklus der Tankstation 1 vorliegen.

### 1.3.1.2 Integriertes Feuerzeug

Das integrierte Feuerzeug 16 ist z. B. ein Vormischbrenner mit Piezozündung, wie bei handelsüblichen Sturmfeuerzeugen bekannt. Das Feuerzeug 16 dient zum Entzünden des Heizstabs 2. Weiter kann es auch als herkömmliches Feuerzeug verwendet werden.

### 1.3.1.3 Multifunktionsdeckel

Der Deckel 19 der Tankstation 1 ist durch seine Formgebung und Kinematik bevorzugt so gestaltet, dass er vier verschiedene Funktionen erfüllt: Heizstab betanken, Heizstab arretieren, Hitzeschild für Heizstabbrenner bereitstellen und Feuerzeug einschalten. Prinzipiell lassen sich auch alle Funktionen des Multifunktionsdeckels durch mehrere Bauteile in verschiedensten Varianten lösen. Der Lösungsansatz "Multifunktionsdeckel" wird jedoch aufgrund des hohen Integrationsgrades von Funktionen als eleganteste und wahrscheinlich kostengünstigste Variante bevorzugt.

Deshalb sollen an dieser Stelle alle vier Funktionen durch den Deckel übernommen werden.

Funktion 1: Heizstab 2 betanken: In geschlossenem Zustand umschließt der Deckel 19 die Spitze des Heizstabes 2 wodurch der Heizstab 2 in seine Aufnahme 18 gedrückt wird, so dass das Ventil 17 der Tankstation 1 am Boden der Aufnahme gasdicht auf das Einlassventil 21 des Heizstabs 2 gepresst wird. Durch das Anpressen öffnen beide Ventile und der Heizstab 2 wird von der Tankstation 1 solange mit Gas gefüllt bis der Druck zwischen Tankstation 1 und Heizstab 2 ausgeglichen ist oder der Deckel 18 geöffnet wird. Der Druckausgleich wird vorzugsweise von einem zischenden Geräusch begleitet. Hört das Geräusch auf, ist der Heizstab voll betankt.

Funktion 2: Heizstab 2 arretieren: Der Rastmechanismus des Deckels 19 ist so ausgelegt, das er mit oder ohne eingelegten Heizstab 2, durch Überdrücken der Tankposition, je nach vorigem Zustand, ein- oder ausrastet. Mit eingelegtem Heizstab 2 wird durch Überdrücken des Deckels 19 der Heizstab 2 betankt.

Funktion 3: Hitzeschild für Brenner: Der Deckel 19 ist so beschaffen, dass er über das nach Gebrauch heiße Ende des Heizstabs 2 (Brennerspitze) geschoben und/oder geklappt wird. Er bildet einen Hitzeschutz, der bei geschlossenem Deckel 19 einen Kontakt mit der Brennerspitze des Heizstabs 2 vermeidet. Weiter ist er in seinen wärmeleitenden Eigenschaften so ausgelegt, dass zum einen die Brennerspitze schnellstmöglich abkühlen kann und zum anderen auf der Deckelaußenseite keine für Menschen unangenehme Temperatur auftritt.

Funktion 4: Feuerzeug einschalten: Im voll geöffneten Zustand wird durch ein Überdrücken des Multifunktionsdeckels 19 das in der Tankstation 1 integrierte Feuerzeug betätigt, d.h. die Gaszufuhr wird frei geschaltet und der Zünder 163 des Feuerzeugs 16 in einer Bewegung getätigt.

### 1.3.1.4 Heizstabaufnahme

Die Aufnahme 18 für den Heizstab 2 ist bevorzugt so gestaltet, dass der Heizstab 2 nur mit seinem mundstückseitigen Ende ohne aufgestecktes Mundstück 3 eingeschoben werden kann.

### 1.3.1.5 Auslassventil

Auf dem Boden der Aufnahme 18 befindet sich das Auslassventil 17 der Tankstation 1, welches z. B. durch Überdrücken oder Schließen des Multifunktionsdeckels 18 bei eingelegtem Heizstab 2 mit diesem gasdicht verbunden wird. Das Auslassventil 17 ist dabei bevorzugt so gestaltet, dass es durch Niederdrücken sich selbst und das Einlassventil 21 des Heizstabs 2 öffnet, so dass Gas aus der Tankstation 1 in den Heizstab 2 strömen kann.

### 2. Heizstab (s. Fig. 1, 2 und 3)

Den nachfolgenden Heizstabausgestaltungen ist gemein, dass sie Luft zum Bilden eines mit Nikotin und Aromen angereicherten Aerosols mittels Verbrennung von Flüssiggas erwärmen. Die Dimensionen des Heizstabs 2 sollten die einer herkömmlichen Zigarette (ohne Filter) nicht signifikant überschreiten.

Dem Brenner 25 ist ein Stofftauscher 26 nachgeschaltet. D.h. die Umgebungsluft wird mit heißem Abgas vermischt. Das Mischverhältnis und somit die Inhalationslufttemperatur am Stofftauscherausgang hängt von der Auslegung des Stofftauschers 26 ab (Verhältnis der Einströmquerschnitte: Stofftauscherabgaseinlass 262 zu Stofftauscherlufteinlass 261).

### 2.1 Bevorzugtes Heizstabkonzept im Detail

### 2.1.1 Hülse

Die Hülse 20 des Heizstabes 2 kann aus einem oder mehreren zylindrischen Teilen bestehen. Das Äußere der Hülse 20 verleiht dem Heizstab 2 sein zigarettenartiges Aussehen und bildet die Haltefläche für den Konsumenten. Sie ist der Hauptkomponententräger des Heizstabs 2. Auf Höhe des Minigastanks 22 ist die Hülse 20 innen so geformt, dass zum einen Warmluftkanäle 27 zwischen Hülse 20 und Minigastank 22 entstehen und zum anderen der Minigastank 22 axial in ihr verschiebbar ist. Am mundstückseitigen Ende hat der Minigastank 22 in seiner axialen Bewegung durch die Hülle 20 einen Anschlag. Im Füllstutzen 220 des Minigastanks 22 ist eine Bohrung 221 vorhanden, die zum einen das Einführen des Heizstabfüllventils der Tankstation 17 bis zum Heizstabfüllventil des Heizstabs 21 ermöglicht und zum anderen einen Strömungskanal bildet. Radial um diese Bohrung 221 sind ringförmig kleine Luftströmungsöffnungen 222 angebracht, die die um den Minigastank 22 angeordneten Warmluftkanäle 27 mit der Bohrung 221 im Füllstutzen 220 verbinden. An der mundstückseitigen Außenfläche der Hülse 20 ist eine Dichtung 201 aufgebracht, die beim Aufschrauben eines Mundstückes 3 auf die Bajonettkupplung des Minigastanks 28 ein Einströmen von Fremdluft verhindert, so dass beim Saugen am aufgeschraubten oder aufgesteckten Mundstück 3 die Luft durch den Heizstab 2 zwangsgeführt wird.

Die Hülse 20 ist Komponententräger und _bevorzugt Funktionspartner für folgende weitere Baugruppen und Bauteile:
a. Regelventil 24
b. Brenner 25

### 2.1.2 Minigastank

Der Minigastank 22 ist die Energiequelle für den Brenner 25 im Heizstab 2 und so dimensioniert, dass das in ihm gespeicherte Flüssiggas für den Konsum eines Mundstücks 3 ausreicht. Die Dauer eines Mundstückkonsums soll der eines Zigarettenkonsums so nah wie möglich kommen, d.h. je nach Gebrauch 3-5 min sein. An seinem mundstückseitigen Ende befindet sich das Heizstabfüllventil 21 zum Betanken des Heizstabs 2 durch die Tankstation 1. Dieses Ende ist z. B. ein Füllstutzen 220 der einen Bajonettverschluss mit dem Mundstück 3 bildet. Der Minigastank 22 ist im Heizstab 2 axial beweglich untergebracht,-so dass beim Aufschrauben/stecken eines Mundstücks 3 der Tank 22 zum Mundstück 3 hin bewegt wird. Am brennerseitigen Ende des Minigastanks 22 ist das Hauptventil 23 untergebracht, ggf. mit einem zwischengeschalteten Druckminderer. Das Hauptventil 23 entspricht im Prinzip einem herkömmlichen Auslassventil eines Feuerzeugs. Im Gegensatz zum Feuerzeug wird das Ventil aber nicht durch Fixieren des Tanks und ziehen der Ventildüse sondern durch Halten der Ventildüse und Ziehen des Tanks 22 betätigt. Wird also ein Mundstück 3 auf den Heizstab 2 aufgeschraubt, gibt das Hauptventil 23 die Gaszufuhr vom Tank 22 zum Reglerventil 24 frei. Zwischen Tank 22 und mundstückseitigem Ende der Hülse 20 kann zusätzlich ein kleines Federelement 223 angebracht werden, falls das Hauptventil 23 keine ausreichende Kraft zum Zurückziehen des Tanks 22 und somit zum Schließen hat, wenn das Mundstück entfernt wird.

### 2.1.3 Reglerventil (s. Fig. 2, 3 und 5)

Das Reglerventil 24 steuert die Gas und Luftzufuhr für den Brenner 25 in Abhängigkeit von der Strömung der Inhalationsluft innerhalb der Hülse 20. Es ist axial beweglich auf dem Mittelteil der Düse zwischen dem Hauptventil 23 des Minigastanks 22 und dem Brenner 25 angeordnet. Außen bildet das Reglerventil 24 mit der Hülse 20 einen schmalen Spalt. In radialer Richtung ist das Reglerventil 24 z. B. mit kleinen Einkerbungen auf der Innenseite zur Regelung des Gasstroms versehen.

Der Spalt zwischen Hülse 20 und Reglerventil 24 ist so ausgelegt, dass das Reglerventil 24 z. B. als Drossel im Luftstrom fungiert. Je nach Druckdifferenz vor und hinter dem Ventil 24 wird es entweder zum Mundstück 3 hingezogen oder von ihm weggedrückt. Das Reglerventil 24 kann zwei relevante Positionen mit folgenden Eigenschaften einnehmen:

- Pilotflamme: Der Regelkörper des Reglerventils 24 befindet sich in Standby-Stellung, wodurch gerade soviel Gas das Ventil passiert, dass die Flamme im Brenner 25 nicht erlischt.

- Betriebsflamme: Der Regelkörper des Reglerventils 24 ist aus der Standby-Stellung durch Sog am Mundstück 3 in Richtung desselben bewegt worden und gibt nun ausreichend Gas für den Brenner 25 frei, sodass dieser ausreichend Brenngas zur Erwärmung der Inhalationsluft abgibt. Wird der Regelkörper aus der Standby-Stellung hinaus in Richtung Mundstück 3 bewegt, so drückt er eine Feder 241 zusammen. Die Feder 241 ist so ausgelegt, dass sie, ohne Sog am Mundstück, den Regelkörper des Ventils 24 in die Standby-Stellung drückt.

Durch verschiedene Geometrien der Öffnungen am Ventil 24 sind verschiedene Kennfelder für den Brenner darstellbar.

### 2.1.4 Brenner

Der Brenner 25 ist bevorzugt ein Flüssiggas-Vormischbrenner, d.h. das brennbare Gemisch aus Gas und durch die Lufteingänge 252 einströmende Luft wird bereits vor dem Düsenaustritt in der Gemischkammer 251 gebildet, sodass eine vollständige Verbrennung des Flüssiggases gewährleistet wird. Zu dieser Art von Brennern werden z. B. Nainen-, Ikari- Matrix- oder Katalytbrenner gezählt und sind teilweise aus Sturmfeuerzeugen und Handgaslötgeräten bekannt.

Typische Nainen-Brenner haben ca. 0,5 cm oberhalb des Düsenauslaßes eine ringförmige Öffnung mit einem so genannten Reaktionsgitter, das durch die Zündflamme erhitzt wird und eine ständige Wiederentzündung des hindurch strömenden Gases bewirkt. Nainen-Brenner bilden oberhalb des Reaktionsgitters eine kegelförmige, kurze, nicht leuchtende, blaue Flamme, die am Fuß einen Durchmesser gleich der Ringöffnung hat.

Zur Verbesserung der Lastwechseleigenschaften zwischen Pilotflamme und Betriebsflamme kann auch die Verwendung eines dreidimensionalen Reaktionsgitters vorgesehen werden.

Weiterhin ist der Brenner 25 optional mit einem Überhitzungsschutz, z.B. in Form eines Bimetallreglers ausgestattet, der z. B. durch Ausdehnung den Regleranschlag steuert und so das Reglerventil 24 bei Überhitzung schließt.

### 2.1.5 Stofftauscher

Der Stofftauscher 26 überträgt durch den Stofftauscherabgaseinlass 262 Abgas vom Brenner 25 zur Inhalationsluft.

Der Stofftauscher 26 ist so gestaltet, dass beim Saugen am Mundstück 3 Abgas mit Umgebungsluft so vermischt wird, dass das entstehende Gemisch die richtige Temperatur zum Lösen der Mixtur aus dem Mundstück 2 besitzt. Dabei muss der Temperaturverlust beim Durchströmen des Heizstabs 2 mit eingerechnet werden.

### 3 Software

Als Software werden alle Bauteile und Stoffe bezeichnet, die nach einmaligem Gebrauch ausgetauscht werden müssen. Die Software besteht aus dem Mundstück 3 und der darin in einem Depot 32 aufgebrachten Mixtur.

### 3.1 Mundstück (Fig. 2, 3, 4, 5, 6 und 7)

Das Mundstück 3 entspricht in seiner Größe in etwa einem Zigarettenfilter, hat jedoch im Gegensatz zu diesem keine Filterfunktion. Im Gegensatz zu einem Filter soll das Mundstück keine Substanzen aus der Inhalationsluft herausziehen, sondern der Inhalationsluft Stoffe hinzusetzen. Alle Teile des Mundstücks 3 bestehen bevorzugt zu 100 % aus biologisch abbaubaren Materialien.

### 3.1.1 Hülse

Die ein- oder mehrteilige Hülse des Mundstücks 3 ist Haltefläche für den Konsumenten und Kontaktfläche mit dem Mund des Konsumenten. Sie ist bevorzugt ein zylindrischer gas- und flüssigkeitsdichter Hohlkörper mit Öffnungen an beiden Enden. Das heizstabseitige Ende ist so geformt, dass die Inhalationsluft ungestört aus den Warmluftausgängen 221 des Heizstabs 2 durch die Heißlufteintrittsöffnungen 30 in das Mundstück 3 strömen kann und dass eine gasdichte Verbindung durch Aufschrauben des Mundstücks 3 auf den Heizstabs 2 entsteht.

### 3.1.2 Depot

Das Depot 32 ist im Prinzip koaxial zur Hülle innerhalb dieser angeordnet und dient der Speicherung der Wirk- und/oder Aromastoff-Mixtur, die auch als Blend bezeichnet wird. Die Affinität des Depotmaterials zur Mixtur muss so hoch sein, dass auch bei Lagerbedingungen mit erhöhter Temperatur so gut wie keine Mixtur vom Depot abgegeben wird. Bei Erreichen der Betriebstemperatur der Inhalationsluft (Warmluft), die das Mundstück durchströmt, muss das Depot 32 jedoch die Mixtur in gefordertem Umfang freisetzen und als Aerosol über die Aerosolaustrittsöffnung 33 dem Benutzer zukommen lassen.

Das Depot 32 im Mundstück 3 kann bei Bedarf auch aus mehreren Kammern bestehen (s. Fig. 7 b).

Um die Unterschiede der Verdampfungstemperaturen von z. B. den Aromen und Nikotin zu berücksichtigen, ist es eventuell notwendig diese in getrennten Depots 32 mit unterschiedlichen Strömungsquerschnitten und/oder unterschiedlichen Eigenschaften des Depotmaterials (Wärmekapazität, Affinität zur aufgegebenen Mixtur, usw.) unterzubringen.

Durch eine Einrastposition z. B. der Bajonettkupplung 28/31 zwischen Mundstück 3 und Heizstab 2 können die Durchströmungsöffnungen von Mundstück 3 und die Warmluftausgänge 221 des Heizstabs 2 in die richtige Position gebracht werden.

### 3.1.3 Mundstückversiegelung

Aus hygienischen Gründen und Gründen der Halt- und Lagerbarkeit kann das Mundstück 3 auch versiegelt werden. Eine Mundstückversiegelung kann z. B. aus zwei kleinen gas- und flüssigkeitsdichten Folien bestehen, die auf die Stirnseiten des Mundstücks geklebt und vor Gebrauch entfernt werden. Zum einfachen Entfernen können die Folien zusätzlich mit einer kleinen Lasche versehen werden.

### 3.2 Wirk- und/oder Aromastoff-Mixtur

Die Mixtur ist der Hauptbestandteil der Rauchsimulation, da sie alle gewünschten Stimulantien enthält, die durch ausreichende Temperatur freigesetzt werden. Die möglichen Inhaltsstoffe gliedern sich in folgende Gruppen.

### 3.2.1 Wirkstoffe

Die Wirkstoffe bestehen in erster Linie aus Nikotin und dessen Derivaten, obwohl andere Stimulantien ebenfalls oder stattdessen eingesetzt werden können, z. B. Koffein und/oder Taurin. Die Menge an Wirkstoff in der Mixtur kann sehr einfach bei der Herstellung dosiert werden und könnte z. B. weniger Nikotin als eine herkömmliche Zigarette bzw. kleine Zigarre enthalten, jedoch bei erreichen der Betriebstemperatur die gleiche Menge, wie eine Zigarette freisetzen. Weiterhin können Vitamine und Mineralien zugesetzt werden.

### 3.2.2 Aroma- oder Geschmacksstoffe

Die Geschmacksstoffe sind hauptsächlich Aromen, die laut Aromenverordnung zugelassen sind. Auch z. B. Zucker kann als Geschmacksstoff zugesetzt werden.

### 3.2.3 Reizstoffe

Die Reizstoffe dienen der Imitation des "Kratzens" im Rachenraum, welches beim Rauchen entsteht. Hier kann z. B. Capsaicin (erzeugt die Schärfe in Lebensmitteln) und/oder andere Reizstoffe in unbedenklichen Mengen Verwendung finden.

### Verflüchtigungsvermittler

Verflüchtigungsvermittler wie Ethanol, Wasser, Polyole oder ähnliche können zur besseren Verdampfung der Mixtur zugesetzt werden.

### Stabilisatoren

Um die Lagerfähigkeit zu erhöhen und/oder unerwünschte Reaktionen zwischen den Bestandteilen der Mixtur zu verhindern können auch diverse Stabilisatoren angewendet werden.

### Weitere Zusatzstoffe

Die Verwendung weiterer Zusatzstoffe ist möglich soweit sie gesundheitlich unbedenklich in den verwendeten Mengen für den Konsumenten sind.

**Tabelle 1: Referenzliste**

| **Nr.** | **Allgemeine Bezeichnung** | **Spezifische Bezeichnung** |
|---|---|---|
| **1** | **Tankstation** | **Tankstation** |
| 10 | Gehäusehülse | |
| 11 | Gasvorratstank | |
| 12 | Kolben | |
| 13 | Erste Kammer | Stickstoffkammer, Druckgaskammer |
| 131 | Überdruckventil | |
| 14 | Zweite Kammer | Butankammer, Flüssiggaskammer |
| 141 | Füllventil | Butankammerfüllventil |
| 15 | Druckminderer | |
| 16 | Brenner | Feuerzeug |
| 161 | Drossel | Feuerzeugdrossel |
| 162 | Brennerventil | Feuerzeugbrennerventil |
| 163 | Zündvorrichtung | Zünder |
| 17 | Auslassventil | Heizstabfüllventil T |
| 18 | Aufbewahrungstasche | Aufnahme |
| 19 | Deckel | |
| **2** | **Heizstab** | **Heizstab** |
| 20 | Hülse | Gehäusehülse |
| 201 | Dichtung | |
| 21 | Füllventil | Heizstabfüllventil H |
| 22 | Gastank | Minigastank |
| 220 | Füllstutzen | |
| 221 | Bohrung | Warmluftausgang |
| 222 | Luftströmungsöffnungen | |
| 223 | | Minitankrückholfeder |
| 23 | Hauptventil | |
| 24 | Reglerventil | Regelkörper |
| 241 | Mittel zur Erzeugung einer Rückstellkraft | Reglerrückholfeder |
| 25 | Brenner | Vormischbrenner |
| 251 | Gemischkammer | |
| 252 | Gemischkammerlufteinlass | |
| 26 | Stofftauscher | |
| 261 | Stofftauscherlufteinlass | |
| 262 | Stofftauscherabgaseinlass | |
| 27 | Warmluftkanäle | |
| 28 | Kupplung | Bajonettkupplung H |
| **3** | **Mundstück** | **Mundstück** |
| 30 | Heißlufteintrittsöffnung | |
| 31 | Kupplung | Bajonettkupplung M |
| 32 | Wirk- und/oder Aromastoffdepot | |
| 33 | Aerosolaustrittsöffnung | |

## Patentansprüche

1. Verfahren zur Verflüchtigung von Wirk- und/oder Aromastoffen zwecks Abgabe eines inhalierbaren Aerosols, wobei Verbrennungsgase eines brennbaren Gases, welches vorzugsweise mit Luftüberschuss verbrannt wird, teilweise oder vollständig, gegebenenfalls vermischt mit Umgebungsluft, durch einen Wirk- und/oder Aromastoff-Depot geführt werden und wobei eine gewünschte Temperatur durch den Anteil an Verbrennungsgasen und ggf. durch das Mischungsverhältnis dieser Verbrennungsgase mit Umgebungsluft wählbar ist.

2. Verfahren nach Anspruch 1, wobei die Einstellung des Anteils an Verbrennungsgasen, bzw. des Mischungsverhältnisses dieser Verbrennungsgase mit Umgebungsluft durch Regelung des Mengenflusses an brennbarem Gas erfolgt.

3. Verfahren nach Anspruch 2, wobei die Reglung des Mengenflusses an brennbarem Gas mittels eines durch einen Sog erzeugten Unterdrucks und/oder Luftstroms erfolgt.

4. Vorrichtung zur Ausführung des Verfahrens nach einem der Ansprüche 1 bis 3, umfassend
➢ ein einen Wirk- und/oder Aromastoff-Depot (32) enthaltendes Mundstück (3),
➢ einen Heizstab (2) umfassend
eine Gehäusehülse (20) mit einem oder mehreren Lufteingängen und einem oder mehreren mundstückseitigen Warmluftausgängen,
ein Füllventil (21) zum Befüllen eines Gastanks (22) mit einem brennbaren Gas, vorzugsweise Propan- oder Butangas,
ein Reglerventil (24) zur gesteuerten Abgabe des Gases aus dem Gastank (22) an einen Brenner (25) und
einen Stofftauscher (26) zur Erwärmung der Luft durch die mittels des Brenners (25) erzeugte Hitze,
wobei das Mundstück (3) mit dem Heizstab (2), in Form und Abmessungen einer Zigarette oder Zigarre, zur Abgabe eines inhalierbaren Aerosols, lösbar verbunden ist und die Steuerung des Reglerventils (24) mittels eines durch einen Sog am Mundstück (3) erzeugten Unterdrucks und/oder Luftstroms erfolgt.

5. Vorrichtung nach Anspruch 4, wobei das Reglerventil (24) einen Regelkörper umfasst, sowie Mittel zur Erzeugung einer Rückstellkraft (241), wobei der Regelkörper (24) einen ersten minimal offenen, sowie einen zweiten offenen Zustand aufweist und wobei der Regelkörper mittels des erzeugten Unterdrucks und/oder Luftstroms aus dem ersten minimal offenen Zustand entgegen einer Rückstellkraft in den zweiten offenen Zustand versetzbar ist und die Mittel zur Erzeugung einer Rückstellkraft derart gewählt sind, dass sie den Regelkörper in den ersten minimal offenen Zustand zurücksetzen können.

6. Vorrichtung nach Anspruch 5, wobei der Regelkörper (24) in dem zweiten offenen Zustand einen Strömungsquerschnitt freigibt der abhängig vom Unterdruck und/oder Luftstrom variabel zwischen einem minimal offenen bis ganz offenen Zustand steuerbar ist.

7. Vorrichtung nach einem der Ansprüche 4 bis 6, umfassend zusätzlich ein Hauptventil (23) zwischen Gastank (22) und Reglerventil (24) welches durch Verbinden des Mundstücks (3) mit dem Heizstab (2) geöffnet und durch Ablösen des Mundstücks (3) wieder geschlossen wird.

8. Vorrichtung nach einem der Ansprüche 4 bis 7, wobei ein oder mehrere zu dem oder den Warmluftausgängen führende Warmluftkanäle (27) koaxial um den Gastank (22) angeordnet sind.

9. Vorrichtung nach einem der Ansprüche 4 bis 8, in der das Wirk- und/oder Aromastoff-Depot (32) als Wirkstoff Nikotin und ggf. Aromastoffe, sowie ggf. weitere Zusatz- und Hilfsstoffe, wie Reizstoffe, Stabilisatoren, Verflüchtigungsvermittler, usw. enthält.

10. Vorrichtung nach Anspruch 9, wobei der oder die Wirkstoff(e), ggf. Aromastoff(e), sowie ggf. weitere Zusatz- und Hilfsstoffe in mehreren Kammern einzeln und/oder gemischt vorliegen.

11. Vorrichtung nach einem der Ansprüche 4 bis 10, in der ein Stofftauscher (26) eingesetzt wird und ein Kohlenmonoxid-Oxydationskatalysator, z. B. ein Hopkalit-Katalysator, diesem Stofftauscher (26) nachgeschaltet ist.

12. Vorrichtung nach einem der Ansprüche 4 bis 11, weiterhin umfassend
➢ eine Tankstation (1) zum wiederabnehmbaren Verbinden und Befüllen des Heizstabs (2) umfassend
einen Gasvorratstank (11) dessen Volumen das Mehrfache des Volumens des Gastanks (22) des Heizstabs (2) beträgt und
ein form- und/oder kraftschlüssig mit dem Füllventil (21) des Heizstabs (2) verbindbares Auslassventil (17).

13. Vorrichtung nach Anspruch 12, wobei die Tankstation (1) Mittel zur Aufbewahrung eines Heizstabs (2) aufweist, umfassend eine das Auslassventil (17) zumindest teilweise umschließende Aufbewahrungstasche (18) zur Aufnahme eines mundstückseitigen Teils des Heizstabs (2), sowie einen umsetzbaren Deckel (19) der in einer geschlossenen Stellung die entgegengesetzte Seite des Heizstabs (2) überdeckt.

14. Vorrichtung nach einem der Ansprüche 12 oder 13, wobei die Tankstation (1) zusätzlich eine Zündvorrichtung (163) enthält.

15. Vorrichtung nach einem der Ansprüche 12 bis 14, wobei die Tankstation (1) weiterhin ein Brennerventil (162) und einen Brenner (16) umfasst.

16. Vorrichtung nach einem der Ansprüche 12 bis 15, wobei die Tankstation (1) weiterhin ein Füllventil (141) zum Befüllen des Gasvorratstanks (11) umfasst.

17. Vorrichtung nach einem der Ansprüche 12 bis 16, wobei die Tankstation (1) einen Gasvorratstank (11) umfasst, der durch einen entlang einem Verstellweg axial beweglichen Kolben (12) in eine mit einem inerten Gas, vorzugsweise Stickstoff, gefüllte erste Kammer (13), und eine mit brennbarem Gas, vorzugsweise Propan- oder Butangas, gefüllte zweite Kammer (14) unterteilt ist, wobei der Druck in der ersten mit inertem Gas gefüllten Kammer (13) über den gesamten Verstellweg des Kolbens (12) ausreicht, um das Gas in der mit brennbarem Gas gefüllten Kammer in flüssiger Phase zu halten.

18. Vorrichtung nach Anspruch 17, dessen mit inertem Gas gefüllte Kammer (13) über ein Überdruckventil (131) mit der Umgebung verbunden ist.

19. Vorrichtung nach Anspruch 17 oder 18, dessen mit brennbarem Flüssiggas gefüllte Kammer (14) über einen Druckminderer (15) mit einem Auslassventil (17) und einem Brennerventil (162) verbunden ist.

## Claims

1. A method for volatilising active and/or aroma substances for the purpose of releasing an inhalable aerosol, wherein combustion gases of a flammable gas, which is preferably combusted with an excess of air, are passed partially or entirely, optionally mixed with ambient air, through an active and/or aroma substance depot and wherein a desired temperature is selectable by the proportion of combustion gases and optionally by the mixing ratio of said combustion gases with ambient air.

2. A method according to claim 1, wherein the setting of the proportion of combustion gases or the mixture ratio of these combustion gases with ambient air is effected by regulating the mass flow of flammable gas.

3. A method according to claim 2, wherein regulation of the mass flow of flammable gas is effected by means of a reduced pressure and/or stream of air generated by suction.

4. A device for implementing the method according to any one of claims 1 to 3, comprising
➢ a mouthpiece (3) containing an active and/or aroma substance depot (32),
➢ a heating member (2) comprising
a housing sleeve (20) with one or more air inlets and one or more hot air outlets at the mouthpiece end,
a filling valve (21) for filling a gas tank (22) with a flammable gas, preferably propane or butane gas,
a regulating valve (24) for the controlled release of gas from the gas tank (22) to a burner (25) and
a mass transfer exchanger (26) for heating the air by the heat generated by means of the burner (25), wherein the mouthpiece (3) is detachably connected to the heating member (2), in the shape and dimensions of a cigarette or cigar, for release of an inhalable aerosol, and control of the regulating valve (24) is effected by means of a reduced pressure and/or stream of air generated by suction on the mouthpiece (3).

5. A device according to claim 4, wherein the regulating valve (24) comprises a regulating member and means for producing a restoring force (241), wherein the regulating member (24) comprises a first minimally open state and a second open state and wherein the regulating member may be displaced by means of the generated reduced pressure and/or stream of air against a restoring force from the first minimally open state into the second open state and the means for producing a restoring force are selected such that they are capable of returning the regulating member to the first minimally open state.

6. A device according to claim 5, wherein the regulating member (24) in the second open state opens a flow section which, depending on the reduced pressure and/or stream of air, is variably controllable between a minimally open and a fully open state.

7. A device according to any one of claims 4 to 6, additionally comprising a main valve (23) between the gas tank (22) and regulating valve (24) which is opened by connecting the mouthpiece (3) to the heating member (2) and is closed again by detaching the mouthpiece (3).

8. A device according to any one of claims 4 to 7, wherein one or more hot air channels (27) leading to the hot air outlet(s) are arranged coaxially around the gas tank (22).

9. A device according to any one of claims 4 to 8, in which the active and/or aroma substance depot (32) contains nicotine as the active substance and optionally aroma substances, and optionally further additives and auxiliaries, such as irritants, stabilisers, volatilisation aids, etc.

10. A device according to claim 9, wherein the active substance(s), optional aroma substance(s), and optional further additives and auxiliaries are present individually and/or mixed in a plurality of chambers.

11. A device according to any one of claims 4 to 10, in which a mass transfer exchanger (26) is used and a carbon monoxide oxidation catalyst, for example a hopcalite catalyst, is arranged downstream of said mass transfer exchanger (26).

12. A device according to any one of claims 4 to 11, furthermore comprising
➢ a fuelling station (1) for detachable connection and filling of the heating member (2) comprising
a gas storage tank (11), the volume of which is a multiple of the volume of the gas tank (22) of the heating member (2) and an outlet valve (17) connectable by form-fit and/or force-fit to the filling valve (21) of the heating member (2).

13. A device according to claim 12, wherein the fuelling station (1) comprises means for storing a heating member (2), comprising a storage cavity (18) which at least partially encloses the outlet valve (17) for accommodating a mouthpiece-end part of the heating member (2), together with a movable lid (19) which in a closed position covers the opposing end of the heating member (2).

14. A device according to either one of claims 12 or 13, wherein the fuelling station (1) additionally contains an ignition device (163).

15. A device according to any one of claims 12 to 14, wherein the fuelling station (1) furthermore comprises a burner valve (162) and a burner (16).

16. A device according to any one of claims 12 to 15, wherein the fuelling station (1) furthermore comprises a filling valve (141) for filling the gas storage tank (11).

17. A device according to any one of claims 12 to 16, wherein fuelling station (1) comprises a gas storage tank (11), which is subdivided by a piston (12), which is axially mobile along an adjustment path, into a first chamber (13) filled with an inert gas, preferably nitrogen, and a second chamber (14) filled with a flammable gas, preferably propane or butane gas, wherein the pressure in the first chamber (13) filled with inert gas is sufficient over the entire adjustment path of the piston (12) to keep the gas in the chamber filled with flammable gas in the liquid phase.

18. A device according to claim 17, whose chamber (13) filled with inert gas is connected to the surrounding environment by way of a pressure relief valve (131).

19. A device according to claim 17 or claim 18, whose chamber (14) filled with flammable liquefied gas is connected to an outlet valve (17) and a burner valve (162) by way of a pressure reducer (15).

## Revendications

1. Procédé de volatilisation de substances actives et/ou aromatiques dans le but de libérer un aérosol inhalable, procédé dans lequel les gaz de combustion d'un gaz combustible brûlé de préférence avec excès d'air, sont guidés en partie ou en totalité, le cas échéant mélangés avec de l'air ambiant, à travers un dépôt de substance active et/ou aromatique, et dans lequel une température visée peut être choisie par la proportion de gaz de combustion et, le cas échéant, par le rapport de mélange desdits gaz de combustion avec l'air ambiant.

2. Procédé selon la revendication 1, dans lequel le réglage de la proportion de gaz de combustion, respectivement du rapport de mélange desdits gaz de combustion avec l'air ambiant, s'effectue par régulation du débit de gaz combustible.

3. Procédé selon la revendication 2, dans lequel la régulation du débit de gaz combustible s'effectue au moyen d'une dépression et/ou d'un courant d'air produit par une aspiration.

4. Dispositif pour la mise en oeuvre du procédé selon l'une des revendications 1 à 3, comprenant
- un embout buccal (3) contenant un dépôt de substance active et/ou aromatique (32)
- une cartouche chauffante (2) comprenant
une enveloppe (20) formant boîtier, à une ou plusieurs entrées d'air, et à une ou plusieurs sorties d'air chaud du côté de l'embout buccal,
une valve de remplissage (21) pour le remplissage d'un réservoir de gaz (22) avec un gaz combustible, de préférence propane ou butane,
une soupape de réglage (24) pour la libération commandée du gaz provenant du réservoir de gaz (22) à un brûleur (25), et
un échangeur de matière (26) pour l'échauffement de l'air par la chaleur produite au moyen du brûleur (25),
dispositif dans lequel l'embout buccal (3) est assemblé de manière amovible avec la cartouche chauffante (2), sous la forme et les dimensions d'une cigarette ou d'un cigare, pour la libération d'un aérosol inhalable, et dans lequel la commande de la soupape de réglage (24) s'effectue au moyen d'une dépression et/ou d'un courant d'air produit par une aspiration sur l'embout buccal (3).

5. Dispositif selon la revendication 4, dans lequel la soupape de réglage (24) comprend un corps de réglage, ainsi que des moyens permettant de générer une force de rappel (241), dans lequel le corps de réglage (24) présente un premier état d'ouverture minimale ainsi qu'un second état ouvert et dans lequel le corps de réglage (24), au moyen de la dépression et/ou du courant d'air produit, peut passer du premier état d'ouverture minimale, à l'encontre d'une force de rappel, au second état ouvert, et dans lequel les moyens permettant de générer une force de rappel sont tels qu'ils sont capables de remettre le corps de réglage dans le premier état d'ouverture minimale.

6. Dispositif selon la revendication 5, dans lequel le corps de réglage (24), dans le second état ouvert, libère une section d'écoulement apte à être commandée en fonction de la dépression et/ou du courant d'air, d'une façon variable, entre un état d'ouverture minimale jusqu'à un état de pleine ouverture.

7. Dispositif selon l'une des revendications 4 à 6, comprenant en outre une soupape principale (23) entre réservoir de gaz (22) et soupape de réglage (24), qui s'ouvre en assemblant l'embout buccal (3) avec la cartouche chauffante (2) et se referme en détachant l'embout buccal (3).

8. Dispositif selon l'une des revendications 4 à 7, dans lequel un ou plusieurs canaux d'air chaud (27) conduisant au(x) sorties d'air chaud, sont disposés coaxialement autour du réservoir de gaz (22).

9. Dispositif selon l'une des revendications 4 à 8, dans lequel le dépôt de substance active et/ou aromatique (32) contient comme substance active de la nicotine et éventuellement des arômes, ainsi que le cas échéant d'autres additifs et auxiliaires tels que stimulants, stabilisants, accélérateurs de volatilisation, etc.

10. Dispositif selon la revendication 9, dans lequel la ou les substances actives, le cas échéant la ou les substances aromatiques, ainsi que le cas échéant d'autres additifs et auxiliaires sont présents séparés et/ou mélangés dans plusieurs chambres.

11. Dispositif selon l'une des revendications 4 à 10, dans lequel un échangeur de matière (26) est utilisé, et un catalyseur d'oxydation du monoxyde de carbone, p. ex. un catalyseur Hopkalit, est monté en aval dudit échangeur de matière (26).

12. Dispositif selon l'une des revendications 4 à 11, comprenant en outre
- un poste de remplissage (1) pour le raccordement amovible et le remplissage de la cartouche chauffante (2), comprenant
un réservoir de stockage de gaz (11), dont le volume est multiple du volume du réservoir de gaz (22) de la cartouche chauffante (2), et
une soupape de décharge (17) apte à être reliée à la soupape de remplissage (21) de la cartouche chauffante (2) par complémentarité de forme et/ou liaison de force.

13. Dispositif selon la revendication 12, dans lequel le poste de remplissage (1) comporte des moyens de conservation d'une cartouche chauffante (2), comprenant une poche de conservation (18) enveloppant au moins partiellement la soupape de décharge (17) et destinée à recevoir une partie de la cartouche chauffante (2) située du côté de l'embout buccal, ainsi qu'un couvercle déplaçable (19) qui en position fermée recouvre le côté opposé de la cartouche chauffante (2).

14. Dispositif selon l'une des revendications 12 ou 13, dans lequel le poste de remplissage (1) comporte en outre un dispositif d'allumage (163).

15. Dispositif selon l'une des revendications 12 à 14, dans lequel le poste de remplissage (1) comporte également une soupape de brûleur (162) et un brûleur (16).

16. Dispositif selon l'une des revendications 12 à 15, dans lequel le poste de remplissage (1) comporte encore une valve de remplissage (141) permettant de remplir le réservoir de stockage de gaz (11).

17. Dispositif selon l'une des revendications 12 à 16, dans lequel le poste de remplissage (1) comprend un réservoir de stockage de gaz (11), lequel est divisé par un piston (12) mobile axialement le long d'une course de réglage, en une première chambre (13) remplie avec un gaz inerte, de préférence l'azote, et une seconde chambre (14) remplie avec un gaz combustible, de préférence propane ou butane, la pression dans la première chambre (13) remplie de gaz inerte étant sur l'ensemble de la course du piston (12) suffisante pour maintenir le gaz dans la chambre remplie de gaz combustible en phase liquide.

18. Dispositif selon la revendication 17, dont la chambre (13) remplie de gaz inerte communique avec l'environnement, par l'intermédiaire d'une soupape de surpression (131).

19. Dispositif selon la revendication 17 ou 18, dont la chambre (14) remplie de gaz liquide combustible communique avec une soupape de décharge (17) et une soupape de brûleur (162), par l'intermédiaire d'un détendeur (15).
